**Europäisches Patentamt**

**European Patent Office**

**Office européen des brevets**

(19)

(11) Veröffentlichungsnummer: **0 000 491**
**B1**

# (12) EUROPÄISCHE PATENTSCHRIFT

(21) Anmeldenummer: **78100350.4**

(22) Anmeldetag: **11.07.78**

(51) Int. Cl.³: **C 07 C 76/06, C 07 C 79/10**

(54) Verfahren zur Aufarbeitung von bei der Nitrierung von Aromaten erhaltenen Nebenprodukten

(30) Priorität: **19.07.77 DE 2732558**

(43) Veröffentlichungstag der Anmeldung:
**07.02.79 Patentblatt 79/03**

(45) Bekanntmachung des Hinweises auf die Patenterteilung:
**01.10.80 Patentblatt 80/20**

(84) Benannte Vertragsstaaten:
**BE CH DE FR GB LU NL**

(56) Entgegenhaltungen:
**DE - C - 700 642**

(73) Patentinhaber: **BAYER AG**
**Zentralbereich Patente, Marken und Lizenzen**
**D - 5090 Leverkusen 1, Bayerwerk (DE)**

(72) Erfinder: **Thiem, Karl-Werner, Dr.**
**Andreas-Gryphius-Strasse 26**
**D - 5000 Köln 80 (DE)**
**Neumann, Lutz, Dr.**
**Wolfskaul 7**
**D - 5000 Köln 80 (DE)**
**Thelen, Bernd**
**Carl-Duisberg-Strasse 327**
**D - 5090 Leverkusen 1 (DE)**

## Verfahren zur Aufarbeitung von bei der Nitrierung von Aromaten erhaltenen Nebenprodukten

Die vorliegende Erfindung betrifft ein Verfahren zur Aufarbeitung von aromatischen Aldehyden und von nitrosen Gasen, die bei der Nitrierung von Aromaten mit aliphatischen Seitenketten in überschüssiger Salpetersäure als unerwünschte Nebenprodukte auftreten.

Im Falle der Nitrierung von Aromaten, die aliphatische Seitenketten tragen, finden außer der gewünschten Nitrierung auch Oxidationsreaktionen an den Seitenketten als unerwünschte Nebenreaktionen statt. Diese Oxidationsreaktionen können z.B. im Falle der Nitrierung in überschüssiger Salpetersäure zur Bildung von Aldehyden und Carbonsäuren führen. Im Falle der Nitrierung von Toluol in überschüssiger Salpetersäure werden z.B. Benzaldehyd, Benzoesäure und Nitrobenzoesäure als Nebenprodukt gebildet.

Die Carbonsäuren können aus dem Reaktionsgemisch nach Verlassen des Nitrierungsreaktors und anschließender Phasentrennung z.B. durch eine Wäsche mit einer wäßrigen alkalischen Lösung und einer anschließenden Neutralwäsche mit Wasser aus der organischen Phase des Reaktionsgemisches entfernt werden. Der in der organischen Phase verbleibende Aldehyd begleitet aufgrund seines hohen Siedepunktes bei der weiteren Aufarbeitung der organischen Phase durch Rektifikation in einer Kolonne, in der der nicht umgesetzte Aromat und anhaftendes Wasser abgetrennt werden, in mehr oder weniger großem Umfang den Nitroaromaten.

Lediglich bei umwirtschaftlich hohen Rücklaufverhältnissen in der Rektifikationskolonne und demzufolge entsprechend hohem Dampfverbrauch für die Destillation gelingt es, den Sumpfablauf aldehydfrei zu erhalten.

Ein Restgehalt an Aldehyd in dem als Sumpfablauf erhaltenen Nitroaromaten führt aber bei etwa folgenden Verarbeitungsstufen zu besonderen Trennproblemen. Bei der Isomerentrennung, wie beispielsweise im Falle der isomeren Nitrotoluole, kann der restgehalt an Aldehyd dazu führen, daß die spezifikationsgerechte Qualität des gewünschten Nitroaromaten-Isomeren nicht erreicht wird.

Mit den als Nebenreaktion zur Nitrierung ablaufenden Oxidationsreaktionen zu aromatischen Aldehyden und aromatischen Carbonsäuren ist gleichzeitig die Reduktion eines Teils der Salpetersäure zu Stickstoffoxiden mit niederigerer Oxidationszahl als 5 (nitrose Gase) verbunden. Dies führt zu einem unerwünschten Mehrverbrauch an Salpetersäure. Die als Abgas entweichenden nitrosen Gase sind außerdem mit dem nicht umgesetzten Aromaten, dem Nitroaromaten und Salpetersäure entsprechend deren Partialdampfdruck beladen, wodurch eine weitere Ausbeuteverminderung eintritt.

Es wurde ein Verfahren zur Trennung und Aufarbeitung von unerwünschten Nebenprodukten, bestehend aus aromatischen Aldehyden und aus nitrosen Gasen, die bei der Nitrierung von aliphatischen Seitenketten enthaltenden, gegebenenfalls substituierten Aromaten in überschüssiger Salpetersäure durch teilweise Oxidation der Seitenketten auftreten gefunden, das dadurch gekennzeichnet ist, daß man gleichzeitig bei der Rektifikation des Nitroaromaten durch Aufrechterhaltung einer wäßrigen Phase innerhalb der Rektifikationskolonne zusätzlich zur organischen Phase den gegebenenfalls substituierten aromatischen Aldehyd als Azeotrop mit Wasser über dem Kolonnenkopf abdestilliert und nach Oxidation zur entsprechenden Carbonsäure ausschleust und die im Abgas enthaltenen nitrosen Gase in einer Adsorptionskolonne mit einer im Nitrierverfahren anfallenden wäßrigen Phase, die gegebenenfalls Salpetersäure enthalten kann, im Gegenstom in Gegenwart von Sauerstoff in Salpetersäure zurücküberführt.

Die Anwendbarkeit des erfindungsgemäßen Verfahrens schließt sich an die Nitrierung von reaktionsfähigen Aromaten, die eine aliphatische Seitenkette tragen, in überschüssiger Salpetersäure an. Solche Aromaten sind beispielsweise Toluol, Xylol, Ethylbenzol, Isopropylbenzol, 1-Methyl-naphthalin oder 2-Methyl-naphthalin.

Die aus den genannten Verbindungen erhältlichen durch Alkylreste substituierten Nitroaromaten durchlaufen nach Verlassen des Nitrierungsreaktors eine Abscheidungsstufe für die im Überschuß eingesetzte Salpetersäure und eine Salpetersäure-Extraktion. Darauf folgt eine Waschstufe, in der mit Hilfe von wäßriger alkalischer Lösung die in Nebenreaktionen entstandenen sauren Bestandteile, beispielsweise Benzosäure oder substituierte Benzosäure, abgetrennt werden. Nach einer anschließenden Neutralwäsche gelangt die organische Phase mit den gewünschten Nitroaromaten in die Rektifikationskolonne.

Wird nun in der Rektifikationskolonne unterhalb der Zulaufstelle für die gewaschene organische Phase Wasser in einer solchen Menge eingespeist, daß an der Zulaufstelle neben der organischen Phase, die Wasser gelöst enthält, ständig zusätzlich eine wäßrige Phase aufrechterhalten wird, so gelingt es, als Sumpfprodukt die gewünschten Nitroaromaten mit einer Aldehydkonzentration von weniger als 0,005 Gew.-% zu erhalten.

Die Ausbildung der benötigten wäßrigen Phase tritt ab etwa 1 Gew.-% Wasser, bezogen auf den Gesamtproduktzulauf zur Rektifikationskolonne, ein. Die Menge des zusätzlich eingespeisten Wassers ist nach oben unkritisch, sie wird lediglich durch Wirtschaftlichkeitsüberlegungen, z.B. durch die bei höheren Wassermengen steigenden Dampfverbräuche, begrenzt. In geringem Umfang ist die optimale Wassermenge zusätzlich abhängig von der Betriebstemperatur bzw. dem Betriebsdruck der Kolonne aufgrund der temperaturabhängigen Löslichkeit von Wasser in der organischen Phase. Eine Wassermenge von 1 bis 5 Gew.-%, bezogen auf den Gesamtproduktzulauf, wird bevorzugt.

Im Kopfprodukt der Rektifikationskolonne treten folgende Stoffe auf: das erfindungsgemäß eingespeiste Wasser, das in der organischen Phase nach Durchlaufen der letzten Waschstufe gelöst befindliche Wasser, der erfindungsgemäß abzutrennende aromatische Aldehyd, eine kleine Menge nicht umgesetztes Ausgangsprodukt und in geringem Umfang der jeweilige Nitroaromat.

Nach der Trennung des Kopfproduktes in eine anorganische und eine organische Phase wird die anorganische Phase in die Neutralwäsche unmittelbar vor der Rektifikationskolonne zugeführt, während die organische Phase mit dem erfindungsgemäß abgetrennten aromatischen Aldehyd, dem nicht umgesetzten Ausgangsprodukt und dem Nitroaromaten in den Eingangsstrom zum Nitrierungsreaktor zurückgeführt wird.

Beim erneuten Durchlaufen der Nitrierstufe wird dann der erfindungsgemäß abgetrennte aromatische Aldehyd zur zugehörigen aromatischen Carbonsäure oxidiert, die dann ihrerseits in der alkalischen Wäsche und in der Neutralwäsche ausgeschleust werden kann.

Durch die erfindungsgemäße Zugabe von Wasser ergeben sich vorteilhafterweise bei der Rektifikation günstigere Rücklaufverhältnisse und/oder eine geringere Anzahl theoretischer Stufen im Abtriebsteil der Kolonne und damit verbunden geringere Dampfverbräuche als ohne diese Maßnahme.

Diese Umstände seien für den Fall der Nitrotoluol-Rektifikation aufgezeigt:

Enthält der Nitrotoluol-Zulauf zur Rektifikationskolonne beispielsweise 0,5 Gew.-% Benzaldehyd und 4,5 Gew.-% nicht umgesetztes Toluol, so muß ohne die erfindungsgemäße Zugabe von Wasser bei 7 (bzw. 11, bzw. 14) theoretischen Stufen im Abtriebsteil der Rektifikationskolonne ein Rücklaufverhältnis von 23 (bzw. 18, bzw. 15), bezogen auf die eingespeite organische Phase, eingestellt werden, um einen nahezu aldehydfreien Sumpfablauf mit einem Gehalt an Benzaldehyd von kleiner 0,005 Gew.-% zu erhalten. Die hierzu benötigte Dampfmenge beträgt 1190 kg (bzw. 950 kg. bzw. 790. kg), bezogen auf 1000 kg Produktzulauf.

Enthält der Nitrotoluol-Zulauf zur Rektifikationskolonne 1% Benzaldehyd und 4% nicht umgesetztes Toluol, so muß ohne die erfindungsgemäße Zugabe von Wasser bei 9 (bzw. 14, bzw. 18) theoretischen Stufen im Abtriebsteil ein Rücklaufverhältnis von 23 (bzw. 18, bzw. 15) eingestellt werden, um bei gleichen Dampfverbräuchen wie oben den Aldehydspiegel von kleiner 0,005 Gew.-% im Sumpfablauf zu erreichen.

Bei Anwendung des erfindungsgemäßen Verfahrens, d.h. bei Einspeisung einer Wassermenge unterhalb der Kolonnenzulaufstelle für den Produktstrom, werden Rücklaufverhältnisse von 10 bis 2,5 möglich, wenn man beispielsweise 1 bis 5% Wasser, bezogen auf die Menge des Produktstromzulaufes, einspeist. Unter diesen Bedingungen beträgt die zum Aufheizen des Sumpfes notwendige Dampfmenge nur etwa 50% der ohne die erfindungsgemäßen Maßnahmen notwendigen Dampfmenge.

Für die Wirtschaftlichkeit des Verfahrens ist nun der überraschende Befund bedeutsam, daß der entstandene und erfindungsgemäß abgetrennte aromatische Aldehyd gemeinsam mit dem über Kopf der Kolonne abdestillierten nicht umgesetzen Aromat in de Nitrierungsstufe zurückgeführt werden kann. Hierbei wird der aromatische Aldehyd zur entsprechenden Carbonsäure weiteroxidiert, die dann in der alkalischen Wäsche und der folgenden neutralen Wäsche ausgeschleust werden kann.

Diese Weiteroxidation zur Carbonsäure geschieht in einem solchen Maße, daß der Aldehydspiegel im Nitrierungsreaktor nicht über ein gewisses Maß ansteigt. Bei der Herstellung von Nitrotoluol stellt sich beispielsweise eine Aldehydkonzentration zwischen 0,5 und 1,0 Gew.-%, bezogen auf die gesamte organische Phase, ein. Ohne die erfindungsgemäße Weiteroxidation des aromatischen Aldehyds durch Einspeisen in die Nitrierstufe wäre eine aufwendige destillative Trennung des nicht umgesetzten Aromaten von dem in der Nebenreaktion entstandenen aromatischen Aldehyd notwendig.

Durch die als Nebenreaktion verlaufende Oxidation von Alkylresten des eingestzten Aromaten zur Aldehydgruppe oder zur Carboxylgruppe, sowie durch die Oxidation des erfindungsgemäß während der Rektification abgetrennten und erfindungsgemäß in die Nitrierstufe wieder eingespeisten aromatischen Aldehyds zur aromatischen Carbonsäure wird ein Teil der Salpetersäure zu Stickstoffoxiden mit niedrigerer Oxidationszahl als 5 reduziert. Solche Stickstoffoxide mit niedrigerer Oxidationszahl als 5 sind beispielswiese Stickstoffdioxid, Distickstofftetroxid, Stickstoffmonoxid und Distickstofftrioxid. Sie werden insgesamt als nitrose Gase bezeichnet.

Diese nitrosen Gase sind zu einem geringen Teil im Reaktionsgemisch gelöst enthalten, teilweise verlassen sie den Nitrierungsreaktor als Abgas.

Dieses Abgas ist außerdem mit nicht umgesetztem Ausgangsprodukt, mit dem durch die Nitrierung hergestellten Nitroatomen und mit Salpetersäure entsprechend deren Partialdampfdruck beladen.

Erfindungsgemäß werden die nitrosen Gase des Abgases mit einer innerhalb des Nitrierverfahrens anfallenden wäßrigen Phase, die gegebenenfalls Salpetersäure enthalten kann, behandelt und in Salpetersäure zurückgeführt. Selbstverständlich ist es möglich, alle anderen Abgase des Nitrierverfahrens, die ebenfalls nitrose Gase enthalten, in diese Abgasbehandlung einzubeziehen.

Die im Abgas weiter enthaltenen Verbindungen, nämlich der nicht umgesetzte Ausgangsstoff, und der im Nitrierverfahren hergestellte Nitroaromat werden nach der Abgas-Behandlung kondensiert und wieder dem Kreislauf zugeführt.

Ein spezielle Ausführungsform dieser Abgasbehandlung unter Rückgewinnung von Salpetersäure, nicht umgesetztem Ausgangsprodukt und Nitroaromat besteht darin, daß man die Abgasbehandlung in einer Kolonne durchführt, bei der dem von unter zugeführten Abgas von oben im Gegenstrom die wäßrige, gegebenenfalls bereits Salpetersäure enthaltende Phase über eine oder mehrere Zulaufstellen entgegengeführt wird. Dabei kann die Kolonne beispielsweise im unteren Abschnitt mit bereits mehr Salpetersäure enthaltender wäßriger Phase beaufschlagt werden, die beispielsweise bei der Extraktion der organischen Phase mit Wasser oder verdünnter Salpetersäure anfällt.

Der obere Teil der Kolonne kann dann mit Wasser oder mit verdünnter Salpetersäure beaufschlagt werden, um den Wirkungsgrad der Umwandlung der Stickstoffoxide in Salpetersäure zu verstärken.

Die Umwandlung der Stickstoffoxide zu Salpetersäure erfordert die Gegenwart von Sauerstoff und erfolgt vorteilhaft in Gegenwart von Luftsauerstoff. Zweckmäßig erfolgt die Zufuhr von Luftsauerstoff unterhalb der Abgaszuführung in die Kolonne. Auf diese Weise läßt sich ein von gelösten nitrosen Gasen weitgehend freier Ablaufstrom erhalten, der einer Salpetersäurekonzentrierung oder einer anderen geeigneten Verwendung zugeführt werden kann.

Zur Erzielung der notwendigen Verweilzeit zur Oxidation in der Gasphase sollte die Kolonne durch Verweilzeitkammern unterteilt sein. Im übrigen kann die Kolonne beispielsweise mit Füllkörpern oder mit Austauschböden ausgerüstet sein. Die Abgasbehandlung kann aber auch beispielsweise in einer Kesselkaskade mit entsprechenden Begasungsvorrichtungen durchgeführt werden.

Die Absorption der nitrosen Gase und ihre Umwandlung in Salpetersäure verläuft exotherm, so daß eine Kühlung der Reaktionsmischung erforderlich werden kann. Dies kann beispielsweise dadurch geschehen, daß man den Eingangsstrom an wäßriger Phase vorkühlt oder daß man die Reaktionsmischung durch separate Kühlvorrichtungen führt. Als günstigste Arbeitsbedingungen haben sich Temperaturen zwischen 10 und 50°C erwiesen.

Die Reaktion kann bei Normaldruck oder bei leichtem Überdruck bis etwa 3 bar durchgeführt werden. Das Arbeiten unter Druck läßt bei gleicher Leistung kleinere Apparatedimensionen zu.

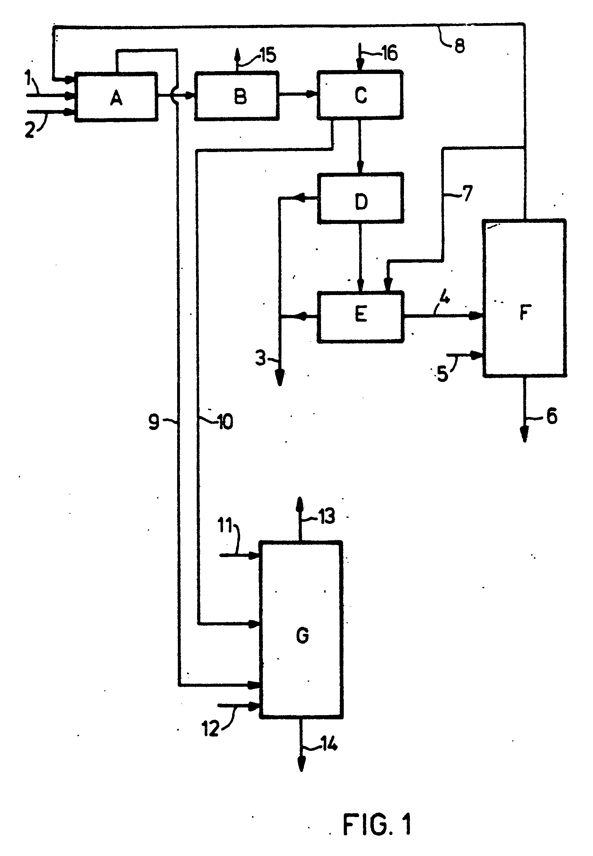

Anhand der Figur 1 sei das Verfahren am Beispiel der Herstellung von Nitrotoluol beschrieben:

In einem geeigneten, kontinuierlich zu betreibenden Nitrierreaktor werden Toluol und Salpetersaure eingespeist. In einem Salpetersäureabscheider wird aus dem Reaktionsgemisch die organische Phase abgetrennt und diese anschließend in einem Salpetersäureextraktor weitgehend von Salpetersäure befreit.

Die organische Phase durchläuft sodann eine alkalische Wäsche und eine neutrale Wäsche, in denen z.B. Benzosäure und Nitrobenzoesäure in Form ihrer Alkalisalze mit dem Abwasser ausgeschleust werden.

Die gewaschene organische Phase wird sodann in eine Rektifikationskolonne eingespeist, die gleichzeitig unterhalb der Einspeisung des organischen Produktes eine Wassereinspeisung besitzt. Das Sumpfprodukt der Rektifikationskolonne ist das gewünschte Nitrotoluol mit einem Benzaldehydgehalt von kleiner 0,005 Gew.-%.

Das Kopfprodukt der Rektifikationskolonne enthält neben einer geringen Menge an Nitrotoluol das Wasser, das nicht umgesetzte Toluol und den auszuschleusenden Benzaldehyd. Diese Kopfprodukt wird in eine anorganische und eine organische Phase getrennt; die anorganische Phase wird in die Waschstufe zurückgeführt, und die organische Phase kann direkt in den Nitrierreaktor zurückgeführt werden.

Die unerwünschte Oxidation der Methylgruppe des Toluols zum Benzaldehyd und der erfindungsgemäße Weiteroxidation des Benzaldehyds zur Benzoesäure zum Zwecke der Ausschleusung führen zur Entstehung nitroser Gase.

Diese nitrosen Gase fallen hauptsächlich als Abluft des Nitrierreaktors an. Sie werden in den unteren Teil einer Absorptionskolonne eingespeist. Unterhalb der Ablufteinspeisung befindet sich eine Zufuhrstelle für atmosphärische Luft, die den zur Umwandlung der Stickstoffoxide in Salpetersäure benötigten Sauerstoff enthält.

In die Mitte der Absorptionskolonne wird eine verdünnte Salpetersäurelösung eingespeist, die bei der Extraktion der Salpetersäure aus der organischen Phase des Nitriergemisches gewonnen wird. Am Kopf der Kolonne wird Wasser eingespeist. Anstelle der Einspeisung von Wasser kann auch die Einspeisung einer sehr verdünnten Salpetersäure treten, die beispielsweise als Kopfprodukt bei der Aufkonzentrierung von unterazeotroper Salpetersäure erhalten wird.

Das ablaufende Sumpfprodukt der Absorptionskolonne wird gemeinsam mit der an der Salpetersäure-Abscheidung gewonnenen überschüssigen Salpetersäure zum erneutenn Einsatz in den Nitrierreaktor aufgearbeitet.

Das erfindungsgemäße Verfahren erlaubt die Abtrennung aromatischer, gegebenenfalls substituierter Aldehyde von gegebenenfalls substituierten aromatischen Nitroverbindungen bei geringerem spezifischen Dampfverbrauch, verglichen mit einer Rektifikation ohne den erfindungsgemäßen Zusatz von Wasser.

Das erfindungsgemäße Verfahren erlaubt die Rückführung des gesamten organischen Anteils am Kopfprodukt der Rektifikationskolonne in die Nitrierstufe und vermeidet dadurch eine aufwendige destillative Trennung des auszuschleusenden aromatischen Aldehyds von den übrigen wieder einsatzfähigen aromatischen Verbindungen, wie beispielsweise die nicht umgesetzten Aromaten und einen geringen im Kopfprodukt der Rektifikationskolonne auftretenden Teil an Nitroaromaten. Dadurch werden Ausbeuteverluste bei den organischen Stoffen vermieden.

Das erfindungsgemäße Verfahren ermöglicht die Umwandlung von Stickstoffoxiden zu Salpetersäure und vermeidet dadurch Ausbeuteverluste bei der Salpetersäure.

### Beispiel 1

In einem Nitrierreaktor, z.B. einer Kaskade von 3 Ringrohrreaktoren, werden kontinuierlich 39,079 kg pro Stunde einer 65,8 gew.-%igen Salpetersäure mit 4,033 kg pro Stunde Toluol, das 0,6 Gew.-% Nitrotoluol und 0,8 Gew.-% Benzaldehyd enthält, bei 70°C und einer Verweilzeit von 6 Minuten umgesetzt. Anschließend wird das Reaktionsgemisch gekühlt und bei 30°C in einer Zentrifuge kontinuierlich in eine anorganische und eine organische Phase getrennt. Die organische Phase (Gehalt an Salpetersäure: 18,2 Gew.-%, an Wasser: 4,9 Gew.-%, an Benzoesäure: 0,3 Gew.-%, an Benzaldehyd: 0,5 Gew.-%, an Nitrobenzoesäure: 0,2 Gew.-%, an Toluol: 2,8 Gew.-%) wird in einer vierstufigen Kaskade, bestehend aus Ringrohrreaktor, Zentrifuge und je drei Wäschern und Scheidern, im Gegenstrom bei 45°C mit 0,746 kg pro Stunde Wasser gewaschen.

Die nach dieser Gegenstromextraktion anfallende wäßrige Phase wird zur Behandlung der anfallenden Abgase verwendet.

Die alkalische Wäsche der organischen Phase besteht aus einer Behandlung mit 3 gew.-%iger wäßriger Natronlauge (2,671 kg pro Stunde). Die anschließende Neutralwäsche erfolgt in einer Kaskade aus gerührten Wäschern und anschließenden Scheidern bei ca. 45°C. Bei diesen beiden Waschstufen werden die in der organischen Phase enthaltenen Säuren als Natriumsalze vollständig mit den Abwasserströmen entfernt.

Die gewaschene organische Phase (Menge: 5,803 kg pro Stunde; 0,6 Gew.-% Benzaldehyd; 3,5 Gew.-% Toluol) wird in einer Bodenkolonne (12 Abtriebsböden; 18 Verstärkungsböden) unter Zugabe von 0,145 kg pro Stunde Wasser bei 160°C Sumpftemperatur und einem reduzierten Druck von ca. 100 Torr Kopfdruck und 130 Torr Sumpfdruck bei einem auf die organische Phase bezogenen Rücklaufverhältnis von 4:1 rektifiziert. Dabei werden 2,175 kg pro Stunde Dampf zum Aufheizen des Sumpfes benötigt. Der Sumpfablauf ist benzaldehydfrei (kleiner 0,005 Gew.-%).

Der organische Teil des Destillats wird nach Abtrennung des Wassers in die Nitrierung zurückgeführt (Menge: 0,259 kg pro Stunde; ca. 77,5% Toluol; 12,5% Benzaldehyd; 10% Nitrotoluol).

Die Ausbeute an Nitrotoluol, bezogen auf das Toluol, beträgt 98,3%.

Die nach der Gegenstromextraktion anfallende wäßrige Phase (2,429 kg pro Stunde mit 53 Gew.-% Salpetersäure) wird zur Behandlung der nitrosen Gase im Abgas in eine 20-stufige Bodenkolonne aur den 15 Boden eingespeist. Der oberste Boden wird mit 0,242 kg pro Stunde einer verdünnten Salpetersäure (2 Gew.-% Salpetersäure) beaufschlagt. Der Abgasstrom (1,010 kg pro Stunde) wird auf den 5. Boden der Kolonne und ein Luftstrom (0,638 kg pro Stunde) auf den 1. Boden der Kolonne gegeben. Die flüssigen Zulaufströme werden auf 10°C vorgekühlt, die Absorption wird bei 15°C und einer Verweilzeit von 6 Minuten betrieben.

Der von organischen Verbindungen freie Abgasstrom der Abgaswäsche (0,799 kg pro Stunde) wird zur Entfernung von durchgeschlagenen Resten der nitrosen Gase eine Absorption mit 25%iger wäßriger Natronlauge zugeführt.

### Beispiel 2

1,000 kg pro Stunde der nach Beispiel 1 erhaltenen gewaschenen organischen Phase werden zur Abtrennung von Toluol und Benzaldehyd kontinuierlich einer Kolonne mit 15 theoretischen Stufen im Abtriebsteil und 10 Stufen im Verstärkungsteil zugeführt, wobei auf den 5. Boden unterhalb des Zulaufs 0,05 kg pro Stunde Wasser zugegeben werden. Die Rektifikation wird mit mit einem auf die organische Phase bezogenen Rücklaufverhältnis von 2,5:1 unter den in Beispiel 1 angegebenen Bedingungen betrieben, wobei 0,425 kg pro Stunde Dampf zum Aufheizen des Sumpfes benötigt werden.

Der Sumpfablauf der Kolonne enthält unter 0,005 Gew.-% Benzaldehyd. Eine ebenso gute Abtrennung des Benzaldehyds erhält man, wenn man 0,05 kg (bzw. 0,025 kg) pro Stunde Wasser zugibt und ein Rücklaufverhältnis von 6:1 (bzw. 10:1) einstellt. Die benötigte Dampfmenge beträgt in diesem Falle 0,600 kg (bzw. 0,675 kg) pro Stunde. Die übrige Führung des Prozesses verläuft wie in Beispiel 1 beschrieben.

### Beispiel 3

Der Nitrierprozeß wird, wie in Beispiel 1 beschrieben, kontinuierlich durchgeführt, aber bei einer Nitriertemperatur von 80°C. Die dabei nach dem Waschen erhaltene organische Phase (Menge: 1,000 kg pro Stunde; 1 Gew.-% Benzaldehyd) wird kontinuierlich einer Kolonne mit 12 theoretischen Stufen im Abtriebsteil und 10 Stufen im Verstärkungsteil zugeführt, wobei auf dem 6. Boden unterhalb des Zulaufs 0,02 kg pro Stunde Wasser zugegeben werden. Die Rektifikation wird bei einem auf die organische Phase bezogenen Rücklaufverhältnis von 10:1 unter den im Beispiel 1 angegebenen Bedingungen betrieben, wobei 0,675 kg pro Stunde Dampf zum Aufheizen des Sumpfes benötigt werden.

Im Sumpfablauf ist gaschromatographisch kein Benzaldehyd mehr nachzuweisen (kleiner 0,005 Gew.-%).

Eine ebenso gute Abtrennung des Benzaldehyds erhält man, wenn man bei 15 theoretischen Stufen im Abtriebsteil einer Zugabe von 0,05 kg pro Stunde Wasser ein Rücklaufverhältnis von 2,5:1 einstellt. Die benötigte Dampfmenge beträgt 0,425 kg pro Stunde.

Die übrige Führung des Prozesses verläuft wie inn Beispiel 1 beschrieben.

*Legende zu Figur 1*

| | |
|---|---|
| A | Nitrierreaktor |
| B | Salpetersäure-Abscheider |
| C | Salpetersäure-Extraktion |
| D | Alkalische Wäsche |
| E | Neutrale Wäsche |
| F | Rektifikationskolonne |
| G | Abgas-Wäsche |

| | |
|---|---|
| 1 | Toluol-Zulauf |
| 2 | Salpetersäure-Zulauf |
| 3 | Abwasser (enthält ausgeschleustes Natriumbenzoat) |
| 4 | gewaschene organische Phase |
| 5 | Wasser-Zulauf |
| 6 | Nitrotoluol, rein |
| 7 | Destillat, wäßrige Phase |
| 8 | Destillat, organische Phase (enthält Benzaldehyd) |
| 9 | Abgas (enthält nitrose Gase) |
| 10 | verdünnte Salpetersäure |
| 11 | Wasser-Zulauf |
| 12 | Luft-Zuführung |
| 13 | Abgas, gereinigt |
| 14 | Salpetersäure zur Wiederverwendung |
| 15 | Salpetersäure zur Wiederverwendung |
| 16 | Wasser (ggf. Salpetersäure enthaltend) |

**Patentansprüche**

1. Verfahren zur Trennung und Aufarbeitung von unerwünschten Nebenprodukten, bestehend aus aromatischen Aldehyden und aus nitrosen Gasen, die bei der Nitrierung von aliphatischen Seitenketten enthaltenden, gegebenenfalls substituierten Aromaten in überschüssiger Salpetersäure durch teilweise Oxidation der Seitenketten auftreten, dadurch gekennzeichnet, daß man gleichzeitig bei der Rektifikation des Nitroaromaten durch Aufrechterhaltung einer wäßrigen Phase innnerhalb der Rektifikationskolonne zusätzlich zur organischen Phase den gegebenenfalls substituierten aromatischen Aldehyd als Azeotrop mit Wasser über den Kolonnenkopf abdestilliert und nach Oxidation zur entsprechenden Carbonsäure ausschleust und die im Abgas enthaltenen nitrosen Gase in einer Adsorptionskolonne in einer im Nitrierverfahren anfallenden wäßrigen Phase, die gegebenenfalls Salpetersäure enthalten kann, im Gegenstrom in Gegenwart von Sauerstoff in Salpetersäure zurücküberführt.

2. Verfahren nach Anspruch 1, dadurch gekennzeichnet, daß als Sauerstoff der Luftsauerstoff verwendet wird.

3. Verfahren nach Anspruch 2, dadurch gekennzeichnet, daß man die Oxidation des aromatischen Aldehyds zur entsprechenden aromatischen Carbonsäure innerhalb des Nitrierungsreaktors vornimmt.

4. Verfahren nach Anspruch 3, dadurch gekennzeichnet, daß man den Luftsauerstoff unterhalb der Abgaszuführung in die Adsorptionskolonne einführt.

**Claims**

1. A process for the isolation and working up of undesired by-products consisting of aromatic aldehydes and nitrous gases, which are obtained during the nitration in excess nitric acid of optionally substituted aromatic compounds containing aliphatic side-chains by partial oxidation of the side-chains, characterized in that at the same time as the rectification of the nitro-aromatic compound, the optionally substituted aromatic aldehyde is distilled off over the head of the column as an azeotrope with water by maintaining an aqueous phase within the rectification column in addition to the organic phase, and after oxidation to the corresponding carboxylic acid is discharged and the nitrous gases contained in the off-gas are reconverted into nitric acid in an adsorption column in an aqueous phase obtained in the nitration process, which can optionally contain nitric acid, in counter-current in the presence of oxygen.

2. The process according to claim 1, characterized in that the oxygen used is atmospheric oxygen.

3. The process according to claim 2, characterized in that the oxidation of the aromatic aldehyde to the corresponding aromatic carboxylic acid is carried out within the nitration reactor.

4. The process according to claim 3, characterized in that the atmospheric oxygen is introduced into the adsorption column below the off-gas feed.

**Revendications**

1. Procédé de séparation et de traitement de sous-produits inopportuns constitués d'aldéhydes aromatiques et de gaz nitreux se formant lors de la nitration d'hydrocarbures aromatiques éventuellement substitués et contenant des chaînes latérales aliphatiques dans de l'acide nitrique en excès par oxydation partielle de ces chaînes latérales, caractérisé en ce que, lors de la rectification de l'hydrocarbure aromatique nitré, on distille simultanément l'aldéhyde aromatique éventuellement substitué sous forme d'un azéotrope avec l'eau par le sommet de la colonne de rectification en maintenant, à l'intérieur de cette dernière, une phase aqueuse en plus de la phase organique, puis on l'exclut après oxydation en un acide carboxylique correspondant et l'on retransforme, en acide nitrique, les gaz nitreux contenus dans le gaz résiduaire dans une colonne d'adsorption avec une phase aqueuse se formant dans le procédé de nitration et pouvant éventuellement contenir de l'acide nitrique et ce, en contre-courant et en présence d'oxygène.

2. Procédé suivant la revendication 1, caractérisé en ce que, comme oxygène, on utilise l'oxygène atmosphérique.

3. Procédé suivant la revendication 2, caractérisé en ce qu'on effectue l'oxydation de l'aldéhyde aromatique en un acide carboxylique aromatique correspondant à l'interieur du réacteur de nitration.

4. Procédé suivant la revendication 3, caractérisé en ce qu'on introduit l'oxygène atmosphérique en dessous du point d'admission du gaz résiduaire dans la colonne d'adsorption.

FIG. 1